# EUROPÄISCHE PATENTSCHRIFT

(11) **EP 3 557 277 B1**
(45) Veröffentlichungstag und Bekanntmachung des Hinweises auf die Patenterteilung: **28.09.2022**
(21) Anmeldenummer: 18168203.0
(22) Anmeldetag: 19.04.2018
(51) Int. Cl.: G01R 33/565, G01R 33/567, G16H 40/63

(54) **VERFAHREN ZUR ERMITTLUNG VON BEWEGUNGSINFORMATIONEN EINES PATIENTEN IN EINER MAGNETRESONANZEINRICHTUNG, MAGNETRESONANZEINRICHTUNG, COMPUTERPROGRAMM UND ELEKTRONISCH LESBARER DATENTRÄGER**
METHOD FOR MONITORING MOVEMENT INFORMATION OF A PATIENT IN A MAGNETIC RESONANCE DEVICE, MAGNETIC RESONANCE DEVICE, COMPUTER PROGRAM AND ELECTRONICALLY READABLE DATA CARRIER
PROCÉDÉ DE DÉTERMINATION DES INFORMATIONS RELATIVES AU MOUVEMENT D'UN PATIENT DANS UN DISPOSITIF DE RÉSONANCE MAGNÉTIQUE, DISPOSITIF DE RÉSONANCE MAGNÉTIQUE, PROGRAMME INFORMATIQUE ET SUPPORT DE DONNÉES LISIBLE PAR VOIE ÉLECTRONIQUE

(43) Veröffentlichungstag der Anmeldung: 23.10.2019
(73) Patentinhaber: Siemens Healthcare GmbH, 91052 Erlangen (DE)
(72) Erfinder: Hölscher, Uvo, 91052 Erlangen (DE); Kartäusch, Ralf, 91058 Erlangen (DE); Horger, Wilhelm, 90571 Schwaig (DE)

(56) Entgegenhaltungen:
- DE-A1-102014 218 901
- US-A1- 2015 157 277

## Beschreibung

Die Erfindung betrifft ein Verfahren zur Ermittlung von eine zyklische Bewegung eines Patienten in einer Magnetresonanzeinrichtung beschreibenden Bewegungsinformationen, wobei die Bewegungsinformationen zur Steuerung der Aufnahme und/oder Auswertung von Magnetresonanzdaten mit der Magnetresonanzeinrichtung verwendet werden und die Bewegungsinformationen durch Auswertung von Bewegungsdaten eines Bewegungsvermessungsmittels ermittelt werden.

Die Magnetresonanzbildgebung stellt ein inzwischen etabliertes Bildgebungsmittel im medizinischen Betrieb dar. Werden Magnetresonanzdaten eines Patienten im Bereich des Torso, insbesondere also in Brust- und/oder Bauchregionen, aufgenommen, besteht die Herausforderung, dass die entsprechenden Aufnahmebereiche durch zyklische Bewegungen des Patienten konkret die Atembewegung und die Herzbewegung, mitbewegt sein können. Dies kann zu Bewegungsartefakten führen.

Verfahren zur Bewegungskorrektur sind beispielsweise aus den Druckschriften DE 102015218106 B4, US 20170016972 A1 oder US 20160091591 A1 bekannt.

Es wurden zwei grundsätzliche, auch in Kombination verwendbare Ansätze zur Vermeidung solcher Bewegungsartefakte vorgeschlagen, die beide auf der Bereitstellung von Bewegungsinformationen über die zyklische Bewegung beruhen. Zum einen wurde vorgeschlagen, solche Bewegungsinformationen bereits bei der Aufnahme der Magnetresonanzdaten selbst zu berücksichtigen, insbesondere, indem die Aufnahme getriggert und/ oder jeweils für Aufnahmezeitfenster der zyklischen Bewegung erfolgt. Nachdem die Atembewegung und die Herzbewegung sich als solche periodischen Bewegungen üblicherweise zumindest ähnlich wiederholen, können mithin Teilabschnitte der jeweiligen Bewegungszyklen bestimmt werden, in denen die Aufnahme von Magnetresonanzdaten erfolgen soll, wobei zu Beginn dieses Teilabschnitts des Bewegungszyklus ein Triggersignal erzeugt wird, um die Aufnahme von Magnetresonanzdaten zu starten, insbesondere für eine als Kennwert aus vorherigen Bewegungsinformationen vorausbestimmte Dauer des Zeitabschnitts (Aufnahmezeitfenster). Ein weiterer Ansatz sieht eine rechnerische Korrektur von Bewegungseffekten bei der Auswertung der Magnetresonanzdaten vor. Hierbei werden die jeweils bei der Aufnahme der Magnetresonanzdaten aktuellen Bewegungsinformationen ebenso aufgezeichnet und später genutzt, um spezielle Magnetresonanzdaten auszuwählen und/oder aufgrund eines Korrekturalgorithmus die Bewegung zu korrigieren.

Wesentlich für diese Ansätze zur Vermeidung bzw. Verminderung von Bewegungsartefakten bei periodischen Bewegungen des Patienten ist, die zyklische Bewegung möglichst zuverlässig und genau zu erfassen. Beispielsweise bei der Atmung besteht die Herausforderung, dass der Patient nicht über die gesamte Dauer der Aufnahme der Magnetresonanzdaten gleichmäßig atmet. Eine weitere Herausforderung hierbei ist, dass durch ein einmalige Verschiebung des Brustkorbs auch nicht periodische Anteile in der Bewegung auftreten, die die Erkennung der periodischen Bewegung erschweren.

Hierbei wurden im Stand der Technik zwei unterschiedliche Methoden bekannt, um Bewegungsinformationen zu ermitteln, nämlich Navigatormessungen und Bewegungssensoren, die am bzw. in unmittelbarer Nähe des Körpers des Patienten angeordnet werden (auch Körpersensoren genannt). Beide Methoden erzeugen eine Bewegungsinformation, insbesondere eine Messgröße, die die Bewegung wiedergibt und aus der Zeitpunkte der Bewegungszyklen abgeleitet werden können.

Navigatoren sind äußerst schnell durchführbare MR-Messungen mit einer Navigatorsequenz, die vor, während (beispielsweise in Aufnahmepausen) und nach der Aufnahme der Magnetresonanzdaten durchgeführt werden können und Informationen über den Bewegungszustand des zu untersuchenden Bildaufnahmebereichs geben. Dabei können derartige Navigatoren beispielsweise als eindimensionale Messungen, die die Position einer von der Bewegung beeinflussten Kante nachvollziehen, realisiert werden, beispielsweise durch Nachverfolgung der Lage des Zwerchfells und/oder der Leberkuppe zur Ermittlung von Bewegungsinformationen zur Atmung. Die Navigatorsequenz kann immer dann ausgespielt werden, wenn die Magnetresonanzsequenz zur Aufnahme der Magnetresonanzdaten gerade pausiert bzw. nicht benötigt wird. Beispielsweise bei einer Atemtriggerung kann in einem Auswertevorgang das Messergebnis der Navigatorsequenz, mithin Bewegungsdaten, ausgewertet werden, um Bewegungsinformationen zu erhalten und immer zur Zeit der vollen Exspiration die Aufnahme von Magnetresonanzdaten für ein kurzes Aufnahmezeitfenster triggern. Nach dem Aufnahmezeitfenster wird wieder die Navigatorsequenz ausgespielt und die Messung erfolgt solange, bis die nächste volle Exspiration aufgrund der Bewegungsinformation festgestellt wird.

Die zweite oben genannte Methode ist die Verwendung von Bewegungssensoren, die ein von der Magnetresonanzbildgebung unabhängiges Messverfahren darstellen und die Bewegung des Körpers des Patienten erfassen können. Beispiele für derartige Bewegungssensoren sind EKG-Messeinrichtungen, ein Atemgurt und/oder ein in eine Lokalspule integrierter Atemsensor. Dabei können die von solchen Bewegungssensoren genutzten Messverfahren unterschiedlich sein und umfassen beispielsweise den Empfang von elektrischen Feldern beim EKG oder die Verstimmung eines Schwingkreises bei einem in eine Lokalspule integrierten Atemsensor.

Ein Vorteil von Bewegungssensoren (Körpersensorik) ist, dass sie unabhängig von den Magnetresonanzmessungen genutzt werden können und damit auch vor, während oder nach der Nutzung der Magnetresonanzeinrichtung aktiv sind und Bewegungsdaten liefern, während der Navigator-Ansatz immer zwischen der Navigatormessung und der Aufnahme der Magnetresonanzdaten hin- und herwechselt. Der Vorteil der Nutzung einer Navigatorsequenz ist, dass die zu vermessenden Navigatorvolumina genauer positionier werden können, so dass mithin eine verlässlichere Messung entsteht.

Beide Methoden, mithin beide Arten von Bewegungsvermessungsmitteln, weisen jedoch auch deutliche Nachteile auf. So erhält man bei einer optimalen Positionierung eines Navigators auf die Leberkuppe zwar die genaueste Atemkurve, jedoch kann die Positionierung fehlerhaft sein. Die Ursache für solche fehlerhaften Positionierungen kann bei einem Bediener liegen, jedoch auch im Auftreten einer Bewegung zwischen einer Magnetresonanzaufnahme, die der Positionierung zugrunde gelegt wird, meist eines Localizers, und dem Zeitpunkt der Positionierung. Der wesentliche Nachteil von Navigatoren ist jedoch, dass diese nicht jederzeit zur Verfügung stehen, sondern nur dann, wenn gerade keine Messung zur Aufnahme der Magnetresonanzdaten mit der Magnetresonanzeinrichtung erfolgt.

Die Signalqualität der Bewegungssensoren ist deutlich abhängig von der Physiologie und der Positionierung des Patienten. Bei einer ungünstigen Positionierung des Patienten bzw. des Bewegungssensors relativ zum Patienten können, beispielsweise bei der Atmung als Bewegung, die Bewegungsdaten, in diesem Fall die Atemkurve, fälschlicherweise die Bauchatmung wiedergeben, welche genau invertiert zur eigentlich gewünschten Atemkurve (Brustatmung) ist. Bei besonders großen/kleinen Patienten treten weitere Probleme mit der Signalqualität auf, die beispielsweise zu gering sein kann, um Triggersignale bei der getriggerten Aufnahme von Magnetresonanzdaten verlässlich zu berechnen. Gerade bei besonders fülligen Personen als Patienten ist der Abstand des Bewegungssensors zum zu vermessenden Bereich gegebenenfalls zu hoch, um die Auswirkungen der Bewegung, insbesondere der Atmung, verlässlich zu messen.

US 2015/0157277 A1 betrifft eine Magnetresonanzeinrichtung und ein Verfahren zur Magnetresonanzbildgebung. Dabei werden sowohl ein externer Monitor, beispielsweise ein Drucksensor, als auch ein interner Monitor, beispielsweise eine Navigatorsequenz, vor der eigentlichen Bildgebung gemessen, und es wird ein Zusammenhang zwischen den Informationen der Monitore ermittelt. Bei der Bilddatenaufnahme wird die Navigatorsequenz nicht genutzt und Gating bzw. Korrekturen werden aufgrund der mittels des Zusammenhangs ermittelten Bewegungsinformation durchgeführt.

DE 10 2014 218 901 A1 betrifft ein Verfahren zur Korrektur von Atemeinflüssen von Aufnahmen eines Untersuchungsobjekts, bei dem durch Korrelation eines internen und eines externen Atemsignals eine Fitfunktion bestimmt wird, so dass die Korrektur aufgrund des externen Atemsignals und der Fitfunktion erfolgen kann.

Der Erfindung liegt daher die Aufgabe zugrunde, eine verlässlichere Möglichkeit zur Ermittlung qualitativ hochwertiger Bewegungsinformationen anzugeben.

Zur Lösung dieser Aufgabe sind bei einem Verfahren der eingangs genannten Art erfindungsgemäß die Merkmale des Anspruchs 1 vorgesehen.

Die gemeinsame Auswertung der Bewegungsdaten der jeweiligen Bewegungsvermessungsmittel kann dabei die Ermittlung der Bewegungsinformation betreffen, jedoch auch eine gegenseitige Beeinflussung des Betriebs des jeweils anderen Bewegungsvermessungsmittels. Bevorzugt wird als Bewegung die Atembewegung vermessen, weswegen sich viele der genannten Ausführungsbeispiele auf die Atmung als periodische bzw. zyklische Bewegung beziehen. In diesem Kontext ist es besonders vorteilhaft, wenn eine die Position des Zwerchfells und/oder einer Leberkante vermessende, insbesondere eindimensionale, Navigatorsequenz verwendet wird und/oder als Bewegungssensor ein Atemgurt und/oder ein in eine Spule integrierter, die Verstimmung eines Schwingkreises vermessenden Atemsensor verwendet wird. Selbstverständlich können auch andere Arten von Navigatoren und Bewegungssensoren im Rahmen der vorliegenden Erfindung eingesetzt werden, beispielsweise kürzlich vorgeschlagene Navigatorsequenzen, die sich auf die Phasenänderung innerhalb eines kleinen Navigatorvolumens der Wirbelsäule beziehen ("phase scout"). Werden andere zyklische Bewegungen, beispielsweise der Herzschlag, betrachtet, wurden ebenso bereits entsprechende Bewegungsvermessungsmittel vorgeschlagen, beispielsweise eine EKG-Messeinrichtung als Bewegungssensor bzw. ultraschnelle eindimensionale Sequenzen als Navigatorsequenz.

Der Erfindung liegt somit, allgemein gesagt, die Idee zugrunde, kumulativ sowohl wenigstens einen Bewegungssensor als auch wenigstens eine Navigatorsequenz einzusetzen, um eine verbesserte Informationsgrundlage hinsichtlich der Bewegung zu erhalten und somit die Qualität der Bewegungserfassung zu verbessern und/oder deren Aufwand zu verringern. Die unterschiedlichen Bewegungskurven, also die Bewegungsdaten, beispielsweise Atemkurven, werden mithin gemeinsam analysiert und/oder verarbeitet und/oder verglichen. Dabei wird ausgenutzt, dass die Bewegungssensoren unabhängig vom Betrieb der Magnetresonanzeinrichtung sind, so dass die Navigatormessung und der Bewegungssensor zur gleichen Zeit aktiv sein können, mithin Bewegungsdaten zum selben aktuellen Bewegungszustand liefern, so dass eine zweckmäßige gemeinsame Auswertung ermöglicht wird. Dabei kann die simultane Nutzung der Navigatorsequenz und des Bewegungssensors sowohl vor der Aufnahme der Magnetresonanzdaten als auch als kontinuierliche Auswertung während der Aufnahme der Magnetresonanzdaten, insbesondere also in einem Gesamtaufnahmezeitraum, erfolgen. In letzterem Fall wird die Navigatorsequenz zur gleichzeitigen Ermittlung von Bewegungsdaten in Aufnahmepausen, was die Magnetresonanzdaten angeht, ausgespielt.

In einer konkreten Ausgestaltung der vorliegenden Erfindung kann vorgesehen sein, dass vor Beginn der Aufnahme der Magnetresonanzdaten Sensordaten des Bewegungssensors mit Navigatordaten der Navigatorsequenz zur Ermittlung wenigstens eines Abweichungswertes verglichen werden. Gleichzeitig aufgenommene Bewegungsdaten beider Bewegungsvermessungsmittel sollten denselben Bewegungszustand beschreiben, so dass ein Vergleich, insbesondere über einen Vergleichszeitraum, zweckmäßig ist, um festzustellen, ob die jeweiligen Bewegungsdaten, die ja die selbe Bewegung beschreiben sollen, hinreichend übereinstimmen und entsprechende Schlussfolgerungen/Maßnahmen hiervon abhängig zu machen.

So sieht eine besonders vorteilhafte Ausgestaltung der vorliegenden Erfindung vor, dass, bei Erfüllung eines den Abweichungswert auswertenden, eine im Wesentlichen vorliegende Übereinstimmung von Navigatordaten und Sensordaten bezüglich der Bewegung anzeigenden Übereinstimmungskriteriums, während eines Gesamtaufnahmezeitraums der Magnetresonanzdaten, also nach Beginn der Aufnahme der Magnetresonanzdaten, die Navigatorsequenz nicht mehr verwendet wird, sondern lediglich Sensordaten als Bewegungsdaten aufgenommen werden. Das bedeutet, wenn in einem kurzen Intervall vor Beginn der Aufnahme der Magnetresonanzdaten, beispielsweise in einem Vergleichszeitraum, die Bewegungsdaten des Bewegungssensors mit den Bewegungsdaten des Navigators korreliert und validiert werden, kann bei hinreichender Übereinstimmung auf die Ausgabe der Navigatorsequenz während der Aufnahme der Magnetresonanzdaten, also im Gesamtaufnahmezeitraum, verzichtet werden. Hieraus lässt sich gegebenenfalls ein deutlicher Zeitgewinn ableiten, nachdem die Ausgabe der Navigatorsequenz gegebenenfalls zusätzliche Teilzeiträume des Gesamtaufnahmezeitraums in Anspruch nimmt. Ferner kann die SAR-Belastung des Patienten vorteilhaft reduziert werden, nachdem aufgrund des niedrigen, wenigstens einen Abweichungswerts gefolgert werden kann, dass auch der Bewegungssensor am Körper des Patienten, also der Körpersensor, hinreichend genaue Bewegungsdaten als Sensordaten liefert, um für die notwendigen Zwecke Bewegungsinformationen abzuleiten.

Eine weitere vorteilhafte Ausgestaltung in diesem Kontext sieht vor, dass bei Erfüllung eines den Abweichungswert, insbesondere die mehreren Abweichungswerte, auswertenden Korrigierbarkeitskriteriums, insbesondere eines einen umgekehrten Verlauf der Sensordaten zu den Navigatordaten aufgrund einer falschen Positionierung des Bewegungssensors anzeigenden Korrigierbarkeitskriteriums, zumindest die danach aufgenommenen Sensordaten zur Übereinstimmung mit den Navigatordaten korrigiert werden. Das bedeutet, wenn eine bestimmte, bevorzugt durch mehrere Abweichungswerte beschriebene Abweichung zwischen den Navigatordaten und den Sensordaten vorliegt, kann, nachdem die Navigatordaten aufgrund ihrer höheren Qualität und der verlässlichen Positionierung des Navigatorvolumens, in dem die Navigatorsequenz misst, die Navigatordaten als verlässlicher angesehen werden, eine Korrekturvorschrift abgeleitet werden, um zukünftige Sensordaten des Bewegungssensors so zu korrigieren, dass sich die Bewegung entsprechend einer Aufnahme mit der Navigatorsequenz (unabhängig davon, ob diese gerade verwendet wird) ergibt. Hierbei wird die Abweichung insbesondere durch Verwendung mehrerer Abweichungswerte nicht nur quantitativ, sondern auch qualitativ beschrieben und analysiert, um die Ableitbarkeit einer Korrekturmaßnahme zu beurteilen und diese dann auch zu ermitteln/anzuwenden, wenn möglich.

Ein derartiger Fall kann insbesondere bei der Atmung als zyklische Bewegung auftreten, nachdem Bewegungssensoren nicht gut zwischen der Brust- und der Bauchatmung unterscheiden können, es aber leicht zu einer Fehlpositionierung, beispielsweise eines Atemgurtes, kommen kann. Navigatoren werden jedoch gezielt auf ein entsprechendes Navigatorvolumen eingeschränkt, so dass hier von verlässlicheren Bewegungsdaten ausgegangen werden kann. Ist beispielsweise ein Atemgurt als Bewegungssensor zu niedrig am Patienten positioniert und misst die Bauchatmung statt der Brustatmung, verläuft diese gerade invertiert, so dass sich aber auch eine Korrekturvorschrift ergibt, durch die die Sensordaten auf den korrekten Atemverlauf korrigiert werden können.

Als weiteres Kriterium kann bevorzugt ein eine zu starke Abweichung der Sensordaten von den Navigatordaten anzeigendes, den Abweichungswert auswertendes Fehlerkriterium eingesetzt werden, bei dessen Erfüllung nach nur noch mit den Navigatordaten als Bewegungsdaten gearbeitet wird. Das bedeutet also, bei einer zu starken, insbesondere nicht korrigierbaren Abweichung der Sensordaten des Bewegungssensors von den Navigatordaten des Navigators kann auf die Bewegungsdaten des Bewegungssensors gänzlich verzichtet werden und im Fehlerfall auf den Navigator zurückgegriffen werden. Dabei ist es in diesem Zusammenhang auch besonders vorteilhaft, eine entsprechende Information an einen Bediener über ein Ausgabemittel auszugeben.

Zur konkreten Nutzung von Bewegungsdaten und entsprechenden Bewegungsinformationen ist es im Stand der Technik bereits bekannt, in einer Anlernphase Kennwerte der Bewegung zu bestimmen, um diese zur Steuerung der Aufnahme der Magnetresonanzdaten einzusetzen. Im Rahmen der vorliegenden Erfindung ist vorgesehen, dass wenigstens ein Aufnahmeparameter für die Aufnahme der Magnetresonanzdaten, insbesondere eine Länge eines Aufnahmezeitfensters und/oder eine Wartezeit zwischen Teilaufnahmeabschnitten und/oder eine Repetitionszeit, in Abhängigkeit eines den zeitlichen Ablauf der Bewegung beschreibenden Kennwerts als Bewegungsinformation gewählt werden. Dabei sind die Kennwerte insbesondere auf einen Zyklus der zyklischen Bewegung bezogen, so dass sie beispielsweise in Bezug auf die Atmung als Bewegung eine durchschnittliche Atemfrequenz, eine Dynamik des Atemsignals und dergleichen beschreiben können. Insbesondere kann auch eine durchschnittliche Atemkurve über die letzten Atemzyklen bestimmt werden, um hieraus die Länge eines Aufnahmezeitfensters zu bestimmen, innerhalb dessen wenig Bewegung stattfindet, beispielsweise nach voller Exspiration. Im Zusammenhang mit solchen Lernphasen existieren durch die Verwendung von zwei unterschiedlichen Bewegungsvermessungsmitteln einige besonders vorteilhafte Möglichkeiten.

So sieht eine bevorzugte Weiterbildung des erfindungsgemäßen Verfahrens vor, dass der Kennwert in einer mehrere Zyklen der Bewegung umfassenden, vor Beginn der Aufnahme der Magnetresonanzdaten liegenden Anlernphase ermittelt wird, wobei in der Anlernphase zumindest teilweise nur der Bewegungssensor verwendet wird. Wird die Messung mit der Navigatorsequenz genutzt, um die Kennwerte zu bestimmen, kann dies erst dann beginnen, wenn die Magnetresonanzeinrichtung einsatzbereit ist, so dass die entsprechende Anlernphase einen signifikanten Zeitraum, beispielsweise 20 Sekunden oder mehr, vor Beginn der Magnetresonanzdaten einnimmt. Erfindungsgemäß wird nun ausgenutzt, dass der Bewegungssensor schon während der Planungsphase der Aufnahme der Magnetresonanzdaten kontinuierlich Bewegungsdaten aufnehmen können, beispielsweise, während der Patient in eine Patientenaufnahme der Magnetresonanzeinrichtung eingefahren wird, während der Bediener die Parameter der Magnetresonanzdatenaufnahme einstellt und/oder Justagemessungen laufen. Bereits in einem Zeitraum, bevor die Durchführung von Navigatormessungen überhaupt möglich ist, können mithin Bewegungssensoren kontinuierlich den Lernvorgang durchführen. Werden nun beide Bewegungsvermessungsmittel kombiniert, kann die Dauer der Anlernphase nach Vorliegen der Aufnahmebereitschaft für die Magnetresonanzdaten und somit auch die Navigatordaten kürzer ausfallen oder sogar komplett entfallen, falls die von dem Bewegungssensor gelieferten Bewegungsinformationen qualitativ ausreichend hoch sind.

Insbesondere kann mithin vorgesehen sein, dass zu einem aus den Sensordaten als Bewegungsdaten abgeleiteten Kennwert und/oder zu den Sensordaten ein Verlässlichkeitswert ermittelt wird, wobei bei einem einen Schwellwert überschreitenden Verlässlichkeitswert auf eine Nutzung der Navigatorsequenz in der Anlernphase verzichtet wird. Auf diese Weise kann eine deutliche Beschleunigung der Vorbereitung der Aufnahme der Magnetresonanzdaten erreicht werden sowie die SAR-Belastung für den Patienten reduziert werden.

Insbesondere dann, wenn durch sonstige Bewegungen des Patienten eine Veränderung eingetreten ist, kann auch wenigstens eine zweite, weitere Anlernphase auf die ursprüngliche, erste Anlernphase folgen. Auch in diesem Zusammenhang kann vorteilhaft das Vorsehen von zwei unterschiedlichen Bewegungsvermessungsmitteln genutzt werden. So kann in diesem Fall vorgesehen sein, dass bei einer aufgrund einer Veränderung der zyklischen Bewegung und/oder einer störenden Zusatzbewegung des Patienten notwendigen zweiten Anlernphase während eines Gesamtaufnahmezeitraums der Magnetresonanzdaten bei unterbrochener Aufnahme derselben sowohl die Sensordaten als auch die Navigatordaten zur Ermittlung des Kennwerts bei verkürzter Dauer der zweiten Anlernphase gegenüber der ursprünglichen Anlernphase verwendet werden. Durch die verwendeten Auswertungsalgorithmen wird in der Auswertung der Bewegungsdaten üblicherweise auch detektiert, ob sich die zyklische Bewegung zu stark verändert hat, beispielsweise die Atemfrequenz stark verändert ist, bzw. ob andere störende Bewegungen aufgetreten sind, beispielsweise der Patient seinen Brustkorb so bewegt hat, dass sich das Navigatorsignal und das Bewegungssensorsignal sprunghaft und nicht periodisch verändert haben. In diesen Situationen wird eine neue, zweite Anlernphase eingefügt, um aktualisierte Kennwerte zu ermitteln. Mit der Kombination der Verwendung einer Navigatorsequenz und eines Bewegungssensors kann nicht nur schneller und verlässlicher erkannt werden, ob eine neue, zweite Anlernphase nötig ist, sondern es stehen für die zweite Anlernphase auch mehr Bewegungsdaten zur Verfügung (nämlich von zwei Bewegungsvermessungsmitteln), so dass sich die Dauer der zweiten Anlernphase reduzieren lässt.

Auch weitergehend kann während des Gesamtaufnahmezeitraums für die Magnetresonanzdaten, wobei einzelne Teilaufnahmeabschnitte durch Aufnahmepausen unterbrochen sein können, wie dargelegt, das Vorhandensein von zumindest zeitweise zwei Datenquellen für Bewegungsdaten vorteilhaft genutzt werden.

Allgemein kann gesagt werden, dass vorzugsweise gleichzeitig aufgenommene Navigatordaten und Sensordaten zu einer gemeinsamen Bewegungsinformation ausgewertet werden können und/oder gegeneinander plausibilisiert werden können, insbesondere jeweils unter Ermittlung eines zugeordneten Konfidenzwertes.

Hierbei wird mithin ausgenutzt, dass sich die Bewegungsdatenmenge zur Analyse der Bewegung verdoppelt. Da der Navigator und der Bewegungssensor voneinander unabhängige und auf verschiedenen Verfahren basierende Informationen liefern, entsteht ein echter Mehrgewinn an Information über die Bewegung. Daher sind Auswertealgorithmen in der Lage, die Bewegungsinformation mit einer höheren Qualität/Verlässlichkeit zu bestimmen. Dabei kann zunächst eine Bewegungsinformation aus beiden Sätzen von Bewegungsdaten zunächst unabhängig bestimmt und dann zusammengeführt werden, möglich ist es aber auch, alle Bewegungsdaten gleichzeitig gemeinsam auszuwerten, um eine Bewegungsinformation zu bestimmen.

In beiden Fällen ist es möglich, einen zugeordneten Konfidenzwert zu ermitteln, indem beispielsweise die Verlässlichkeiten der einzelnen Bewegungsdaten genauso wie Abweichungen der Bewegungsdaten untereinander berücksichtigt werden. Werden Bewegungsinformationen zunächst unabhängig sowohl für die Navigatordaten als auch für die Sensordaten bestimmt, kann eine Plausibilitätsüberprüfung zwischen den beiden Ergebnissen durchgeführt werden, auf der beispielsweise ein Konfidenzmaß als Verlässlichkeitswert basieren kann, das angibt, wie sicher sich der Auswertealgorithmus über sein Ergebnis ist.

Dabei sei allgemein darauf hingewiesen, dass bei der gemeinsamen Auswertung eine gegenseitige Gewichtung von Sensordaten und Navigatordaten stattfinden kann. Dabei kann insbesondere vorgesehen sein, dass die Navigatordaten bei der gemeinsamen Auswertung gleichzeitig aufgenommener Bewegungsdaten stärker gewichtet werden als die Sensordaten, nachdem die Navigatordaten im Allgemeinen als verlässlicher anzusehen sind.

Neben dem Verfahren betrifft die Erfindung auch eine Magnetresonanzeinrichtung, aufweisend eine zur Durchführung des erfindungsgemäßen Verfahrens ausgebildete Steuereinrichtung und einen mit der Steuereinrichtung verbundenen Bewegungssensor. Sämtliche Ausführungen bezüglich des erfindungsgemäßen Verfahrens lassen sich analog auf die erfindungsgemäße Magnetresonanzeinrichtung übertragen, so dass auch mit dieser die bereits genannten Vorteile erhalten werden können. Die Steuereinrichtung kann wenigstens einen Prozessor und/oder wenigstens ein Speichermittel aufweisen. Neben einer Sequenzsteuereinheit zur allgemeinen Steuerung der Vermessung von Magnetresonanzsignalen, welche vorliegend auch zur Aufnahme der Magnetresonanzdaten sowie der Navigatordaten genutzt werden kann, kann die Steuereinrichtung eine modifizierte Auswertungseinheit aufweisen, die als Bewegungsdaten sowohl die Sensordaten als auch die Navigatordaten nutzt, um einen bestmöglichen Nutzen aus den vorhandenen Informationen zu ziehen, wie dies zum Verfahren erläutert wurde.

Ein erfindungsgemäßes Computerprogramm ist beispielsweise direkt in einen Speicher einer Steuereinrichtung einer Magnetresonanzeinrichtung ladbar und weist Programmmittel auf, um die Schritte eines erfindungsgemäßen Verfahrens auszuführen, wenn das Computerprogramm in der Steuereinrichtung der Magnetresonanzeinrichtung ausgeführt wird. Das Computerprogramm kann auf einem erfindungsgemäßen elektronisch lesbaren Datenträger gespeichert sein, welcher mithin darauf gespeicherte elektronisch lesbare Steuerinformationen umfasst, welche zumindest ein erfindungsgemäßes Computerprogramm umfassen und derart ausgestaltet sind, dass sie bei Verwendung des Datenträgers in einer Steuereinrichtung einer Magnetresonanzeinrichtung ein erfindungsgemäßes Verfahren durchführen. Bei dem Datenträger kann es sich insbesondere um einen nichttransienten Datenträger, beispielsweise eine CD-ROM, handeln.

Weitere Vorteile und Einzelheiten der vorliegenden Erfindung ergeben sich aus den im Folgenden beschriebenen Ausführungsbeispielen sowie anhand der Zeichnung. Dabei zeigen:
- Fig. 1: eine Prinzipskizze einer erfindungsgemäßen Magnetresonanzeinrichtung, und
- Fig. 2: einen Ablaufplan eines Ausführungsbeispiels des erfindungsgemäßen Verfahrens.

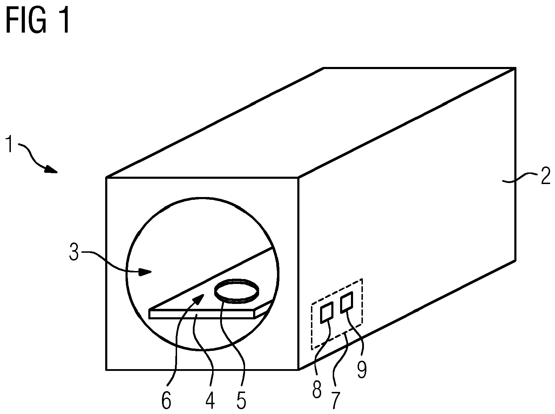

Fig. 1 zeigt eine Prinzipskizze einer erfindungsgemäßen Magnetresonanzeinrichtung 1. Diese weist, wie grundsätzlich bekannt, eine Hauptmagneteinheit 2 auf, in der eine Patientenaufnahme 3 ausgebildet ist, in die mittels einer Patientenliege 4 ein Patient eingefahren werden kann. Die Patientenaufnahme 3 umgebend können, hier aus Übersichtlichkeitsgründen nicht näher gezeigt, eine Hochfrequenzspulenanordnung und eine Gradientenspulenanordnung vorgesehen sein.

Vorliegend auf der Patientenliege 4 gezeigt ist zudem ein Atemgurt 5 als Bewegungssensor 6. Die Magnetresonanzeinrichtung 1 kann auch andere und/oder weitere Bewegungssensoren 6 umfassen, beispielsweise einen in einer Lokalspule verbauten Atemsensor, eine EKG-Messeinrichtung und dergleichen.

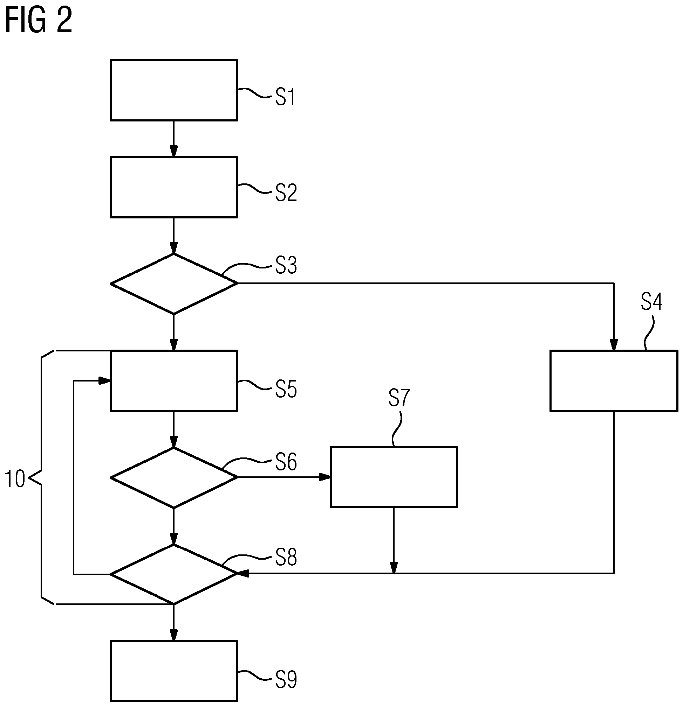

Der Betrieb der Magnetresonanzeinrichtung 1 wird durch eine hier nur angedeutete Steuereinrichtung 7 gesteuert, welche neben anderen Komponenten vorliegend insbesondere eine Sequenzeinheit 8 und eine Auswertungseinheit 9 aufweist, mit deren Hilfe die Steuereinrichtung 7 ausgebildet ist, das erfindungsgemäße Verfahren durchzuführen, welches im Folgenden im Hinblick auf Fig. 2 näher erläutert werden soll.

Dabei wird vorliegend eine Untersuchung eines Patienten im Torsobereich, beispielsweise eine Aufnahme von Magnetresonanzdaten im Bauch- und/oder Brustbereich, konkret in einem speziellen Aufnahmebereich, der der Atmung unterworfen ist, betrachtet. Der Aufnahmebereich ist mithin der Atmung als zyklische (periodische) Bewegung unterworfen. Wie grundsätzlich bekannt, sollen dabei Bewegungsdaten des Patienten aufgenommen werden, um Bewegungsinformationen zu ermitteln, die bei der Aufnahme und bei der Auswertung der Magnetresonanzdaten berücksichtigt werden sollen. Dabei werden zunächst in einer Anlernphase Kennwerte der aktuellen zyklischen Atembewegung ermittelt, um die Dauer eines Aufnahmezeitfensters nach voller Exspiration, zu welchem Zeitpunkt ein Triggersignal erzeugt wird, während der Aufnahme der Magnetresonanzdaten zu ermitteln. Während des Gesamtaufnahmezeitraums, in dem die Magnetresonanzdaten zusammengestellt werden, werden einzelne Aufnahmeabschnitte durch ein entsprechendes Triggersignal bei voller Exspiration getriggert und danach für die bestimmte Dauer des Aufnahmezeitfensters Magnetresonanzdaten aufgenommen, woran sich eine Aufnahmepause anschließt, bis wiederum das Triggersignal vorliegt. Zu allen Magnetresonanzdaten werden Bewegungsdaten oder Bewegungsinformationen aufgezeichnet, so dass diese später auch bei der Auswertung für eine retrospektive Bewegungskorrektur herangezogen werden können.

Nachdem derartige Vorgehensweisen grundsätzlich bekannt sind, soll im Rahmen der Fig. 2 hauptsächlich auf solche Maßnahmen eingegangen werden, die auf der erfindungsgemäßen Nutzung sowohl einer Navigatorsequenz als auch eines Bewegungssensors 6 zur Aufnahme von Bewegungsdaten beruhen.

Nachdem der Patient auf der Patientenliege 4 positioniert wurde, wird ihm nun zunächst nun der Atemgurt 5 angelegt, so dass in einem Schritt S1 bereits zu diesem Zeitpunkt begonnen werden kann, Bewegungsdaten des Atemgurts 5, in diesem Fall also Sensordaten, aufzunehmen. Das bedeutet, bereits während dieser Planungsphase, in der noch keine Magnetresonanzmessung erfolgen kann, liegen bereits Bewegungsdaten vor, so dass zweckmäßigerweise die Anlernphase zur Aufnahme mehrerer Zyklen der Atmung, um den wenigstens einen Kennwert zu bestimmen, bereits im Schritt S1 beginnt.

Die Anlernphase kann im Schritt S2, ab dem Magnetresonanzmessungen möglich sind, vervollständigt werden, indem nun, vor Beginn der Aufnahme der Magnetresonanzdaten, bereits Navigatordaten als weitere Bewegungsdaten mittels der Navigatorsequenz aufgenommen werden. Das bedeutet, im hier dargestellten Ausführungsbeispiel umfassen die zur Ermittlung des wenigstens einen Kennwerts aufgenommenen Bewegungsdaten sowohl Sensordaten als auch Navigatordaten, wobei weniger Navigatordaten aufgenommen werden müssen, mithin das entsprechende Zeitfenster im Schritt S2 verkürzt ist. Bei hoher Qualität der Sensordaten kann auf Navigatordaten in dieser ersten, ursprünglichen Anlernphase auch verzichtet werden, dies ist jedoch weniger bevorzugt, da die Navigatordaten im Schritt S2 auch einem anderen Zweck dienen.

Denn im Schritt S2 findet zudem eine gemeinsame Auswertung der gleichzeitig aufgenommenen Sensordaten und Navigatordaten statt, um die Abweichung zwischen diesen beschreibende Abweichungswerte zu bestimmen. Abweichungswerte können beispielsweise auch beschreiben, ob der Atemgurt 5 zu niedrig am Patienten angeordnet ist, so dass dort die Bauchatmung vermessen wird, welche invertiert zur Brustatmung verläuft, welche bevorzugt durch die Navigatordaten beschrieben wird. In diesem Zusammenhang ist es zweckmäßig, als Navigatorsequenz eine eindimensionale Sequenz zur Bestimmung der Position des Zwerchfells und/oder der Leberkante zu verwenden, wobei die entsprechende Positionierung des Navigatorvolumens auf Grundlage eines Localizers stattfinden kann.

In einem Schritt S3 werden die Abweichungswerte durch mehrere Kriterien ausgewertet. Ist eine besonders gute Übereinstimmung, beschrieben durch ein Übereinstimmungskriterium, gegeben, ist also beispielsweise die Summe des Betrags der Abweichungswerte kleiner als ein Schwellwert, so kann auf hochqualitative Sensordaten geschlossen werden, die es erlauben, auf Navigatormessungen während des Gesamtaufnahmezeitraums der Magnetresonanzdaten gänzlich zu verzichten. Ist demgegenüber ein eine zu starke Abweichung der Sensordaten von den Navigatordaten anzeigendes Fehlerkriterium im Schritt S3 erfüllt, kann in umgekehrter Variante auch vorgesehen sein, lediglich die Navigatordaten als Bewegungsdaten während des Gesamtaufnahmezeitraums zu verwenden. Beide Fälle werden vorliegend zusammenfassend durch einen Schritt S4 abgedeckt, der, nachdem er im Wesentlichen bekannten Vorgehensweisen entspricht, hier nicht näher erläutert werden soll. In beiden Fällen, in denen nur noch eine der Quellen für Bewegungsdaten verwendet wird, wird zweckmäßigerweise eine Information an einen Bediener über einer Ausgabemittel ausgegeben.

Nicht zwangsläufig muss eine starke Abweichung zwischen den Navigatordaten und den Sensordaten jedoch zu einem Ausschluss der Bewegungsdaten führen. Beispielsweise dann, wenn die Sensordaten die Bauchatmung statt der Brustatmung beschreiben, ist es möglich, eine Korrekturmaßnahme aus den die Abweichung auch qualitativ beschreibenden Abweichungswerten zu ermitteln und anzuwenden, so dass die durch die korrigierten Sensordaten beschriebene Bewegung der entspricht, wie sie durch die Navigatormessung beschrieben wird bzw. würde. Im genannten Beispiel kann hierzu beispielsweise eine Invertierung stattfinden. Das bedeutet, im Schritt S3 wird auch ein Korrigierbarkeitskriterium ausgewertet, bei dessen Erfüllung die danach aufgenommenen Sensordaten zur Übereinstimmung mit den Navigatordaten korrigiert werden. Ein entsprechender Korrekturalgorithmus kann durch die Abweichungswerte parametriert werden.

Es wird darauf hingewiesen, dass es durchaus denkbar ist, dass im Schritt S3 keines der drei genannten Kriterien erfüllt ist, woraufhin dann genau wie bei Erfüllung des Korrigierbarkeitskriteriums mit dem Schritt S5 fortgefahren wird, bei dem während des in Fig. 2 ebenso markierten Gesamtaufnahmezeitraums 10 neben den Magnetresonanzdaten sowohl Navigatordaten als auch Sensordaten aufgenommen werden und, insbesondere wenigstens hinsichtlich der Triggerung, gemeinsam ausgewertet werden. Dazu sei im Allgemeinen noch angemerkt, dass beispielsweise Bewegungsinformationen durch gemeinsame Betrachtung von Navigatordaten und Sensordaten bestimmt werden können, es aber auch möglich ist, zunächst unabhängig die Navigatordaten und die Sensordaten zu einer eigenen Bewegungsinformation, beispielsweise einem Triggersignal, auszuwerten, wobei beide Bewegungsinformationen dann zusammengeführt werden. In beiden Fällen ist es möglich, einen Konfidenzwert zu bestimmen, wobei vorliegend zweckmäßigerweise die Navigatordaten höher gewichtet werden können als die Sensordaten, da eine höhere Genauigkeit angenommen werden kann; allerdings liegen Navigatordaten selbstverständlich nur außerhalb von Teilaufnahmeabschnitten, in denen Magnetresonanzdaten aufgenommen werden, vor.

In den Schritten S4 und S5 werden selbstverständlich auch spezielle aus den Kennwerten der Atembewegung abgeleitete Aufnahmeparameter als Bewegungsinformation genutzt, konkret vorliegend die Dauer des Aufnahmezeitfensters.

Aufgrund einer zu starken Veränderung der Atembewegung bzw. des zusätzlichen Auftretens sonstiger, störender Bewegungen, beispielsweise eines vom Patienten vorgenommenen Hebens oder Senkens des Brustkorbs, kann eine weitere, zweite Anlernphase notwendig sein, was im Schritt S6 überprüft wird. Auch diese Überprüfung lässt sich qualitativ hochwertiger und verlässlicher aufgrund des Vorliegens von Sensordaten und Navigatordaten als Bewegungsdaten durchführen. Ist eine zweite Anlernphase notwendig, wird diese im Schritt S7 durchgeführt, insbesondere mit verkürzter Zeitdauer gegenüber der ersten Anlernphase, nachdem auch hier sowohl Sensordaten als auch Navigatordaten als Bewegungsdaten zur Ermittlung der aktualisierten Kennwerte und somit der aktualisierten Aufnahmeparameter verwendet werden können.

In einem Schritt S8 wird überprüft, ob bereits alle Magnetresonanzdaten aufgenommen wurden, wobei, falls dies der Fall ist, zum Schritt S9 fortgefahren wird, in dem die Magnetresonanzdaten ausgewertet werden. Beispielsweise für eine retrospektive Bewegungskorrektur können auch hier die Sensordaten und die Navigatordaten als Bewegungsdaten bzw. hieraus gemeinsam abgeleitete Bewegungsinformationen berücksichtigt werden.

Es sei noch darauf hingewiesen, dass es im Schritt S6 auch denkbar ist, zu überprüfen, ob eine Bewegung des Patienten oder des Bewegungssensors 6 stattgefunden hat, die die weitere Ermittlung von Bewegungsdaten allgemein oder zumindest Bewegungsdaten des Bewegungssensors 6 nicht mehr vertretbar erscheinen lässt, woraufhin dann eine entsprechende Ausgabe an einen Bediener an dem bereits erwähnten Ausgabemittel erfolgen kann.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen, der durch die Ansprüche definiert wird .

## Patentansprüche

1. Verfahren zur Ermittlung von eine zyklische Bewegung eines Patienten in einer Magnetresonanzeinrichtung (1) beschreibenden Bewegungsinformationen, wobei die Bewegungsinformationen zur Steuerung der Aufnahme und optional zur Auswertung von Magnetresonanzdaten mit der Magnetresonanzeinrichtung (1) verwendet werden und die Bewegungsinformationen durch Auswertung von Bewegungsdaten eines Bewegungsvermessungsmittels ermittelt werden, wobei als Bewegungsvermessungsmittel sowohl eine mit der Magnetresonanzeinrichtung (1) durchzuführende Navigatorsequenz als auch ein an dem Patienten angeordneter Bewegungssensor (6) verwendet werden, wobei die mittels der Navigatorsequenz aufgenommenen Navigatordaten als Bewegungsdaten und die von dem Bewegungssensor (6) gemessenen Sensordaten als Bewegungsdaten wenigstens teilweise gemeinsam ausgewertet werden, **dadurch gekennzeichnet, dass** wenigstens ein Aufnahmeparameter für die Aufnahme der Magnetresonanzdaten in Abhängigkeit eines den zeitlichen Ablauf der Bewegung beschreibenden Kennwerts als Bewegungsinformation gewählt wird.

2. Verfahren nach Anspruch 1, **dadurch gekennzeichnet, dass** als Bewegung die Atembewegung vermessen wird.

3. Verfahren nach Anspruch 2, **dadurch gekennzeichnet, dass** eine die Position des Zwerchfells und/oder einer Leberkante vermessende, insbesondere eindimensionale, Navigatorsequenz verwendet wird und/oder als Bewegungssensor (6) ein Atemgurt (5) und/oder ein in eine Spule integrierter, die Verstimmung eines Schwingkreises vermessender Atemsensor verwendet wird.

4. Verfahren nach einem der vorangehenden Ansprüche, **dadurch gekennzeichnet, dass** vor Beginn der Aufnahme der Magnetresonanzdaten Sensordaten des Bewegungssensors (6) mit Navigatordaten der Navigatorsequenz zur Ermittlung wenigstens eines Abweichungswertes verglichen werden.

5. Verfahren nach Anspruch 4, **dadurch gekennzeichnet, dass** bei Erfüllung eines den Abweichungswert auswertenden, eine im Wesentlichen vorliegende Übereinstimmung von Navigatordaten und Sensordaten bezüglich der Bewegung anzeigenden Übereinstimmungskriteriums während eines Gesamtaufnahmezeitraums (10) der Magnetresonanzdaten die Navigatorsequenz nicht verwendet wird, sondern lediglich Sensordaten als Bewegungsdaten aufgenommen werden.

6. Verfahren nach Anspruch 4 oder 5, **dadurch gekennzeichnet, dass** bei Erfüllung eines den Abweichungswert, insbesondere die mehreren Abweichungswerte, auswertenden Korrigierbarkeitskriteriums, insbesondere eines einen umgekehrten Verlauf der Sensordaten zu den Navigatordaten aufgrund einer falschen Positionierung des Bewegungssensors (6) anzeigenden Korrigierbarkeitskriteriums, zumindest die danach aufgenommenen Sensordaten zur Übereinstimmung mit den Navigatordaten korrigiert werden.

7. Verfahren nach einem der Ansprüche 4 bis 6, **dadurch gekennzeichnet, dass** bei Erfüllung eines eine zu starke Abweichung der Sensordaten von den Navigatordaten anzeigenden, den Abweichungswert auswertenden Fehlerkriteriums danach nur noch mit den Navigatordaten als Bewegungsdaten gearbeitet wird.

8. Verfahren nach einem der vorangehenden Ansprüche, **dadurch gekennzeichnet, dass** als der wenigstens eine Aufnahmeparameter eine Länge eines Aufnahmezeitfensters und/oder eine Wartezeit zwischen Teilaufnahmeabschnitten und/oder eine Repetitionszeit verwendet wird.

9. Verfahren nach einem der vorangehenden Ansprüche, **dadurch gekennzeichnet, dass** der Kennwert in einer mehrere Zyklen der Bewegung umfassenden, vor Beginn der Aufnahme der Magnetresonanzdaten liegenden Anlernphase ermittelt wird, wobei in der Anlernphase zumindest teilweise nur der Bewegungssensor (6) verwendet wird.

10. Verfahren nach Anspruch 9, **dadurch gekennzeichnet, dass** zu einem aus den Sensordaten als Bewegungsdaten abgeleiteten Kennwert und/oder zu den Sensordaten ein Verlässlichkeitswert ermittelt wird, wobei bei einem einen Schwellwert überschreitenden Verlässlichkeitswert auf eine Nutzung der Navigatorsequenz in der Anlernphase verzichtet wird.

11. Verfahren nach Anspruch 9 oder 10, **dadurch gekennzeichnet, dass** bei einer aufgrund einer Veränderung der zyklischen Bewegung und/oder einer störenden Zusatzbewegung des Patienten notwendigen zweiten Anlernphase während eines Gesamtaufnahmezeitraums (10) der Magnetresonanzdaten bei unterbrochener Aufnahme derselben sowohl die Sensordaten als auch die Navigatordaten zur Ermittlung des Kennwerts bei verkürzter Dauer der zweiten Anlernphase gegenüber der ursprünglichen Anlernphase verwendet werden.

12. Verfahren nach einem der vorangehenden Ansprüche, **dadurch gekennzeichnet, dass** gleichzeitig aufgenommene Navigatordaten und Sensordaten zu einer gemeinsamen Bewegungsinformation ausgewertet werden und/oder gegeneinander plausibilisiert werden, insbesondere jeweils unter Ermittlung eines zugeordneten Konfidenzwertes.

13. Verfahren nach einem der vorangehenden Ansprüche, **dadurch gekennzeichnet, dass** die Navigatordaten bei der gemeinsamen Auswertung gleichzeitig aufgenommener Bewegungsdaten stärker gewichtet werden als die Sensordaten.

14. Magnetresonanzeinrichtung (1), aufweisend eine zur Durchführung eines Verfahrens nach einem der vorangehenden Ansprüche ausgebildete Steuereinrichtung (7) und einen mit der Steuereinrichtung (7) verbundenen Bewegungssensor (6).

15. Computerprogramm, welches die Schritte eines Verfahrens nach einem der Ansprüche 1 bis 13 durchführt, wenn das Computerprogramm auf einer Steuereinrichtung (7) einer Magnetresonanzeinrichtung (1) gemäß Anspruch 14 ausgeführt wird.

16. Elektronisch lesbarer Datenträger, auf dem ein Computerprogramm nach Anspruch 15 gespeichert ist.

## Claims

1. Method for determining movement information describing a cyclical movement of a patient in a magnetic resonance device (1), wherein the movement information is used to control the recording and optionally to evaluate magnetic resonance data with the magnetic resonance device (1) and the movement information is determined by evaluating movement data of a movement measuring means, wherein both a navigator sequence to be carried out with the magnetic resonance device (1) and also a movement sensor (6) arranged on the patient are used as movement measuring means, wherein the navigator data recorded by means of the navigator sequence is evaluated as movement data and the sensor data measured by the movement sensor (6) is evaluated at least partly together as movement data, **characterised in that** at least one recording parameter for the recording of the magnetic resonance data is selected as movement information as a function of a characteristic value describing the temporal course of the movement.

2. Method according to claim 1, **characterised in that** the breathing movement is measured as the movement.

3. Method according to claim 2, **characterised in that** a in particular one-dimensional navigator sequence which measures the position of the diaphragm and/or a liver edge is used and/or a breathing strap (5) and/or a breathing sensor integrated into a coil and measuring the detuning of an oscillating circuit is used as the movement sensor (6).

4. Method according to one of the preceding claims, **characterised in that** before starting the recording of the magnetic resonance data, sensor data of the movement sensor (6) is compared with navigator data of the navigator sequence in order to determine at least one variance value.

5. Method according to claim 4, **characterised in that** when one of the compliance criterion evaluating the variance value and indicating a substantially present compliance of navigator data and sensor data with respect to the movement is fulfilled during an overall recording time (1) of the magnetic resonance data, the navigator sequence is not used, but only sensor data is recorded as movement data.

6. Method according to claim 4 or 5, **characterised in that** when a correctability criterion evaluating the variance value, in particular the number of variance values, in particular a correctability criterion indicating a reverse course of the sensor data to the navigator data on account of an incorrect position of the movement sensor (6) is fulfilled, at least the subsequently recorded sensor data is corrected for compliance with the navigator data.

7. Method according to one of claims 4 to 6, **characterised in that** when a fault criterion indicating an excessively strong variance of the sensor data from the navigator data and evaluating the variance value is fulfilled, only navigator data is then worked with as movement data.

8. Method according to one of the preceding claims, **characterised in that** a length of a recording time frame and/or a waiting time between sub recording sections and/or a repetition time is used as the at least one recording parameter.

9. Method according to one of the preceding claims, **characterised in that** the characteristic value is determined in a learning phase comprising a number of cycles of the movement and occurring before the start of the recording of the magnetic resonance data, wherein only the movement sensor (6) is used at least partially in the learning phase.

10. Method according to claim 9, **characterised in that** a reliability value is determined in respect of a characteristic value derived from the sensor data as movement data and/or in respect of the sensor data, wherein with a reliability value which exceeds a threshold value, use of the navigator sequence in the learning phase is dispensed with.

11. Method according to claim 9 or 10, **characterised in that** with a second learning phase which is required on account of a change in the cyclical movement and/or an interfering additional movement of the patient during an overall recording time frame (10) of the magnetic resonance data when the recording of the same is interrupted, both the sensor data and also the navigator data are used to determine the characteristic value with a shortened duration of the second learning phase compared with the original learning phase.

12. Method according to one of the preceding claims, **characterised in that** navigator data and sensor data relating to a shared item of movement information and recorded simultaneously are evaluated and/or made plausible with one another, in particular in each case by determining an assigned confidence value.

13. Method according to one of the preceding claims **characterised in that**
with the shared evaluation of simultaneously recorded movement data the navigator data is weighted more significantly than the sensor data.

14. Magnetic resonance device (1), having a control facility (7) embodied for carrying out a method according to one of the preceding claims and a movement sensor (6) connected to the control facilty (7).

15. Computer program, which carries out the steps of a method according to one of claims 1 to 13, when the computer program is executed on a control facility (7) of a magnetic resonance facilty (1) according to claim 14.

16. Electronically readable data carrier, on which a computer program according to claim 15 is stored.

## Revendications

1. Procédé de détermination d'informations de mouvement, décrivant un mouvement cyclique d'un patient dans un dispositif (1) de résonnance magnétique, dans lequel on utilise les informations de mouvement pour la commande de l'enregistrement et éventuellement pour l'analyse de données de résonnance magnétique par le dispositif (1) de résonnance magnétique, et on détermine les informations de mouvement en analysant des données de mouvement d'un moyen de mesure du mouvement, dans lequel, comme moyen de mesure du mouvement, on utilise à la fois une séquence de navigateur à effectuer par le dispositif (1) de résonnance magnétique et un capteur (6) de mouvement posé sur le patient, dans lequel on analyse conjointement au moins en partie les données de navigateur enregistrées au moyen de la séquence de navigateur comme données de déplacement et les données de capteur mesurées par le capteur (6) de mouvement comme données de mouvement, **caractérisé en ce que** l'on choisit comme information de mouvement au moins un paramètre d'enregistrement des données de résonnance magnétique en fonction d'une valeur caractéristique décrivant la variation du mouvement en fonction du temps.

2. Procédé suivant la revendication 1, **caractérisé en ce que** l'on mesure comme mouvement le mouvement respiratoire.

3. Procédé suivant la revendication 2, **caractérisé en ce que** l'on utilise une séquence de navigateur, notamment unidimensionnelle, mesurant la position du diaphragme et/ou d'un bord du foie et/ou on utilise comme capteur (6) de mouvement une sangle (5) respiratoire et/ou un capteur de respiration intégré dans une bobine et mesurant le désaccord d'un circuit oscillant.

4. Procédé suivant l'une des revendications précédentes, **caractérisé en ce qu'**avant le début de l'enregistrement des données de résonnance magnétique, on compare des données du capteur (6) de mouvement à des données de la séquence de navigateur pour la détermination d'au moins une valeur d'écart.

5. Procédé suivant la revendication 4, **caractérisé en ce que**, lorsqu'est satisfait un critère d'accord, analysant la valeur d'écart et indiquant un accord sensiblement présent entre des données de navigateur et des données de capteur en ce qui concerne le mouvement, on n'utilise pas, pendant un laps de temps (10) total d'enregistrement des données de résonnance magnétique, la séquence de navigateur, mais seulement des données du capteur comme données de mouvement.

6. Procédé suivant la revendication 4 ou 5, **caractérisé en ce que**, lorsqu'est satisfait un critère de possibilité de correction analysant la valeur d'écart, notamment les plusieurs valeurs d'écart, notamment un critère de possibilité de correction indiquant une variation inverse des données du capteur par rapport aux données du navigateur en raison d'un mauvais positionnement du capteur (6) de mouvement, on corrige au moins les données du capteur enregistrées ensuite pour la mise en accord avec les données du navigateur.

7. Procédé suivant l'une des revendications 4 à 6, **caractérisé en ce que**, si un critère d'erreur analysant la valeur d'écart et indiquant un écart trop grand des données du capteur aux données du navigateur est satisfait, on ne travaille ensuite qu'avec les données du navigateur comme données de mouvement.

8. Procédé suivant l'une des revendications précédentes, **caractérisé en ce que** l'on utilise, comme le au moins un paramètre d'enregistrement, une longueur d'une fenêtre de temps d'enregistrement et/ou un temps d'attente entre des parties d'enregistrements partielles et/ou un temps de répétition.

9. Procédé suivant l'une des revendications précédentes, **caractérisé en ce que** l'on détermine la valeur caractéristique dans une phase d'apprentissage comprenant plusieurs cycles du mouvement et se trouvant avant le début de l'enregistrement des données de résonnance magnétique, dans lequel, dans la phase d'apprentissage, on utilise au moins en partie seulement le capteur (6) de mouvement.

10. Procédé suivant la revendication 9, **caractérisé en ce que** l'on détermine une valeur de fiabilité par rapport à une valeur caractéristique, déduite des données du capteur comme données de mouvement, et/ou par rapport aux données du capteur, dans lequel, si une valeur de fiabilité dépasse une valeur de seuil, on renonce à une utilisation de la séquence de navigateur dans la phase d'apprentissage.

11. Procédé suivant la revendication 9 ou 10, **caractérisé en ce que**, lors d'une deuxième phase d'apprentissage nécessaire en raison d'une modification du mouvement cyclique et/ou d'un mouvement supplémentaire perturbateur du patient, on utilise, pendant un laps de temps (10) total d'enregistrement des données de résonnance magnétique, alors que leur enregistrement est interrompu, à la fois les données du capteur et les données du navigateur, pour la détermination de la valeur caractéristique pendant une durée de la deuxième phase d'apprentissage écourtée par rapport à la phase d'apprentissage à l'origine.

12. Procédé suivant l'une des revendications précédentes, **caractérisé en ce que** l'on analyse et/ou l'on rend vraisemblable les unes par rapport aux autres, en une information commune de mouvement, des données du navigateur et des données du capteur enregistrées en même temps, notamment respectivement avec détermination d'une valeur de confiance associée.

13. Procédé suivant l'une des revendications précédentes, **caractérisé en ce que** l'on pondère, plus fortement que les données du capteur, les données du navigateur, lors de l'analyse commune de données de mouvement enregistrées en même temps.

14. Dispositif (1) de résonnance magnétique, comportant un dispositif (7) de commande, constitué pour effectuer un procédé suivant l'une des revendications précédentes, et un capteur (6) de mouvement relié au dispositif (7) de commande.

15. Programme d'ordinateur, qui effectue les stades d'un procédé suivant l'une des revendications 1 à 13, lorsque le programme d'ordinateur est réalisé sur un dispositif (7) de commande d'un dispositif (1) de résonnance magnétique suivant la revendication 14.

16. Support de données, déchiffrable électroniquement, sur lequel est mis en mémoire un programme d'ordinateur suivant la revendication 15.
